# EUROPEAN PATENT SPECIFICATION

(11) **EP 1 157 016 B1**
(45) Date of publication and mention of the grant of the patent: **08.10.2003**
(21) Application number: 00907458.4
(22) Date of filing: 03.03.2000
(51) Int. Cl.: C07D 295/088

(54) **A PROCESS FOR THE PREPARATION OF 2- 2- 4-(DIPHENYLMETHYL)-1-PIPERAZINYL]ETHOXY ACETIC ACID COMPOUNDS OR SALTS THEREOF**
VERFAHREN ZUR HERSTELLUNG VON 2-(2(4-(DIPHENYLMETHYL)-1-PIPERAZINYL)ETHOXY)ESSIGSÄUREDERIVATEN ODER IHREN SALZEN
PROCEDE DE PREPARATION DE COMPOSES D'ACIDE ACETIQUE 2- 2- 4-(DIPHENYLMETHYL)-1-PIPERAZINYL]ETHOXY OU DE SELS DE CEUX-CI

(30) Priority: 04.03.1999 DK 990303
(43) Date of publication of application: 28.11.2001
(73) Proprietor: A/S GEA Farmaceutisk Fabrik, 2650 Hvidovre (DK)
(72) Inventor: FISCHER, Erik, DK-2300 Copenhagen S (DK); TREPPENDAHL, Svend, Peter, DK-2830 Virum (DK)
(74) Representative: Nyeng, Joergen
(86) International application number: DK0000090
(87) International publication number: WO00052000

(56) References cited:
- GB-A- 2 225 320
- STN International, File CAPLUS, CAPLUS accession no. 1995:665177, Document no. 123:55923, Warszawskie Zaklady Farmaceutyczne "Polfa", Pol.: "Method of obtaining 2-(2-(4-(4-chlorophenyl) phenylmethyl)-1-piperazinyl)ethoxy)acetic acid (cetirizine) and its dihydrochloride"; XP002120695 & PL 163415 B1 19940331

## Description

### FIELD OF THE INVENTION

The present invention relates to a new and improved process for the preparation of 2-{2-[4-(diphenylmethyl]-1-piperazinyl]ethoxy}acetic acid compounds or salts thereof and to novel 2-{2-[4-(diphenylmethyl]-1-piperazinyl]-ethoxy}acetaldehyde compounds and dialkylacetals thereof which are intermediates in the process.

### BACKGROUND OF THE INVENTION

In light of the versatility of 2-{2-[4-[(4-chlorophenyl)phenylmethyl]-1-piperazinyl]ethoxy}acetic acid in the form of its dihydrochloride - also known by the generic name of cetirizine - as a powerful drug for the treatment of allergic diseases, a new, cheap, easy to perform and high yielding process for its preparation is in great need.

EP Patent No. 58 146 (UCB, S.A.) describes a process for the synthesis of i.a. 2-{2-[4-(diphenylmethyl)-1-piperazinyl]ethoxy}acetic acid compounds which comprises reacting a 1-(diphenylmethyl)piperazine compound with methyl (2-chloroethoxy)acetate or 2-(2-chloroethoxy)acetamide to form a methyl 2-{2-[4-(diphenylmethyl)-1-piperazinyl]-ethoxy}acetate or a 2-{2-[4-(diphenylmethyl)-1-piperazinyl]ethoxy}acetamide, respectively. The formed methyl ester or acetamide is then subjected to basic hydrolysis followed by acidification and isolation of the free carboxylic acid which is eventually transformed into its dihydrochloride. The main problem concerning this approach is that the overall yield of cetirizine dihydrochloride based on 1-[(4-chlorophenyl)phenylmethyl]piperazine is only 10.6 %.

A development of this process is the subject of EP Patent Application No. 801 064 (UCB, S.A.) according to which 1-[(4-chlorophenyl)phenylmethyl]piperazine is reacted with 2-chloroethoxyacetic acid in an inert solvent in the presence of an acid acceptor such as potassium carbonate and optionally in the presence of a small amount of an alkali metal iodide to accelerate the reaction. The reaction is generally effected by heating at between 80 and 150 °C during several hours. The process is stated to be more simple and easy to perform than that of EP Patent No. 58 146, but nothing is said about the yield.

A process similar to that of EP Patent No. 58 146 is presented in GB Patent No. 2 225 321 (UCB, S.A.) according to which 1-[(4-chlorophenyl)phenylmethyl]piperazine is reacted with a 2-haloethoxyacetonitrile. The resulting 2-{2-[4-[(4-chlorophenyl)phenylmethyl]-1-piperazinyl]-ethoxy}acetonitrile is then subjected to either basic or acidic hydrolysis resulting in 2-{2-[4-[(4-chlorophenyl)-phenylmethyl]-1-piperazinyl]ethoxy}acetic acid in 65.6 % yield overall. As this process involves the use of 1.4 equivalents of haloethoxyacetonitrile, which is not commercially available, and furthermore requires column chromatography for purification, it is not feasible for large scale production.

GB Patent No. 2 225 320 (UCB, S.A.) describes a process wherein 2-{4-[(4-chlorophenyl)phenylmethyl]-1-piperazinyl}ethanol is reacted with an alkali metal haloacetate and potassium tert. butoxide. By a rather complicated procedure involving continuous addition of the haloacetate and the tert. butoxide a yield of 55.5 % of 2-{2-[4-[(4-chlorophenyl)phenylmethyl]-1-piperazinyl]ethoxy}acetic acid is obtained. Although the yield is better than in the process of EP Patent No. 58 146, the method and especially the continuous addition of reactants during the reaction is somewhat difficult to handle. In addition, alkali metal haloacetate and potassium tert. butoxide are used in a 1.66 fold excess, which adds to the cost of the process.

PL Patent No. 163 415 (Warszawskie Zaklady Farmaceutyczne "POLFA") describes a process for the synthesis of 2-{2-[4-[(4-chlorophenyl)phenylmethyl]-1-piperazinyl]ethoxy}acetic acid using the same starting materials as in GB Patent No. 2 225 320, but a different set of reaction conditions. By refluxing a two-phase system consisting of 2-{4-[(4-chlorophenyl)phenylmethyl]-1-piperazinyl}ethanol and an alkali metal haloacetate in toluene as one phase, and solid alkali metal hydroxide as the other phase, a yield of 60 % of 2-{2-[4-[(4-chlorophenyl)phenylmethyl]-1-piperazinyl]ethoxy}acetic acid was isolated. Although this procedure gives a somewhat better yield than the one used in GB Patent No. 2 225 320, alkali metal haloacetate is still used in rather large quantities, e.g. 1.5 fold excess.

The international Patent Application WO 97/37982 (UCB, S.A.) describes certain piperazinylethoxyacetic acid derivatives and their utilization for the preparation of i.a. cetirizine by reaction with diphenylmethylhalogenides. By refluxing (4-chlorophenyl)phenylmethylchloride and potassium 2-(1-piperazinyl)ethoxyacetate in acetonitrile for 16 hours cetirizine is obtained in a yield of 48.5 %.

The international Patent Application WO 98/02425 (APOTEX INC.) describes the synthesis of 2-{2-[4-[(4-chlorophenyl)phenylmethyl]-1-piperazinyl]ethoxy}acetic acid by oxidation of 2-{2-[4-[(4-chlorophenyl)phenylmethyl]-1-piperazinyl]ethoxy}ethanol with an oxidation agent. Two embodiments are described therein. The first one comprises reacting 2-{2-(4-[(4-chlorophenyl)phenylmethyl]-1-piperazinyl]ethoxy}ethanol with Jones' reagent: chromium trioxide and sulfuric acid. A very tedious workup process is therefore needed in order to remove any chrome from the product. In addition, the use of large amounts of chrome in a given synthesis can only be considered a serious environmental hazard. The other embodiment involves the use of oxygen and platinum dioxide on carbon. As oxygen in the presence of an organic solvent (here dioxane) constitutes a serious safety hazard, this preparation is probably not feasible for the synthesis of larger amounts of cetirizine than the 0.51 g produced in this example.

Thus, there is a need for a new economical and high yielding process for the synthesis of cetirizine.

### SUMMARY OF THE INVENTION

The present invention provides a process for the manufacture of compounds of the general formula I: 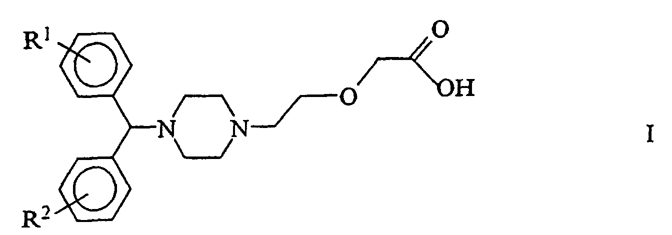 wherein R¹ and R² independently represent a hydrogen atom, a halogen atom or a trifluoromethyl radical. The halogen atom is Br, Cl, F or I.

According to the process of the invention a 2-[4-(diphenylmethyl)-1-piperazinyl]ethanol (II) is first reacted with a 2-substituted acetaldehyde dialkylacetal (III) in the presence of a proton acceptor in an inert solvent: 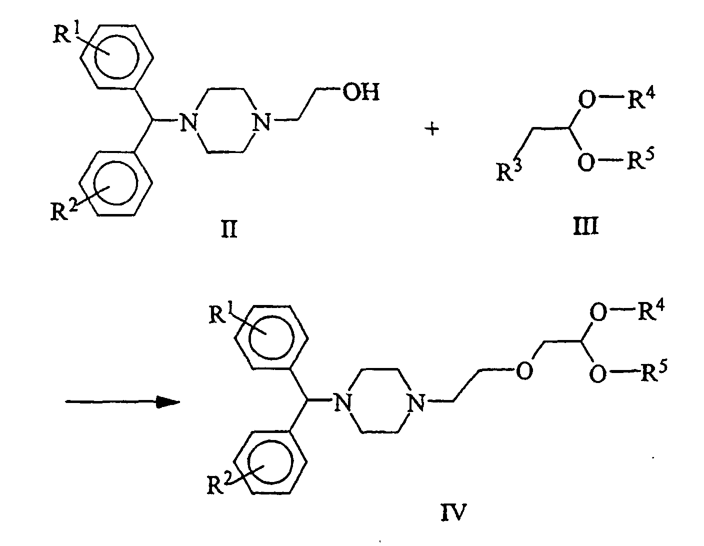 where R¹ and R² are as defined above, R³ represents a leaving group e.g. Cl, Br, I, or a sulfuric ester group; an R⁴ and R⁵ independently represent an alkyl radical of 1-4 carbon atoms, or R⁴ and and R⁵ together form an alkylene chain of 2-4 carbon atoms.

As the proton acceptor sodium hydride seems to work best, but in principle any proton acceptor known to those skilled in the art can be used, e.g. other alkali metal hydrides, alkali metal hydroxides, alkali metal alkoxides such as sodium or potassium tert butoxide and free alkali metals. As the solvent any chemically inert solvent such as aliphatic and aromatic hydrocarbons, ethers, amides and alcohols can be used. Examples of such solvents are hexane, toluene, dimethoxyethane (DME), tetrahydrofurane (THF), dimethylformamide (DMF) and tert-butanol. The reaction is generally carried out at a temperature between room temperature and the boiling point of the chosen solvent.

The resulting diphenylmethylpiperazinoethoxyacetaldehyde dialkylacetal (IV) is then hydrolysed to the corresponding aldehyde, catalysed by a proton donor e.g. hydrochloric acid or formic acid, whereafter the aldehyde is oxidised to the acid (I) by means of a suitable oxidation agent. If desired, the acid (I) is converted into a salt thereof.

Suitable oxidation agents are e.g. reagents based on metals such as chromium, manganese, nickel, selenium etc., oxygen as well as oxo acids of halogens, e.g. sodium hypochlorite, or, even better, peroxides, e.g. hydrogen peroxide, potassium peroxodisulfate, potassium peroxomonosulfate ("Oxone"®) and peracids like m-chloroperoxybenzoic acid. The reaction is usually conducted in an alcoholic/aqueous solution or an aqueous suspension, but any solvent known to those skilled in the art can be used.

The diphenylmethylpiperazinoethoxyacetaldehyde dialkylacetal compounds of the general formula IV above and the corresponding free aldehydes (IVa) which are intermediates in the process of the invention are novel compounds.

The most interesting compound to be prepared by the process of the invention is at present 2-{2-[4-[(4-chlorophenyl)phenylmethyl]-1-piperazinyl]ethoxy}acetic acid in the form of its dihydrochloride known by the generic name of cetirizine.

### DETAILED DESCRIPTION OF THE INVENTION

The first step in the synthesis of cetirizine comprises reacting 2-{4-[(4-chlorophenyl)phenylmethyl]-1-piperazinyl}ethanol with a haloacetaldehyde derivative. Quite a lot of haloacetaldehyde derivatives are now commercially available, but for large scale processes, a stable, reactive, and fairly cheap haloacetaldehyde derivative must be used. The far cheapest one is chloroacetaldehyde dimethylacetal, but when this derivative was used, very long reaction times were required, and furthermore the quality of the resulting product was rather low. We have found that this reaction gives both excellent yield and excellent quality of the resulting product when the leaving group is bromine, and for the sake of reducing cost, we have thus used bromoacetaldehyde diethylacetal.

The choice of solvent for this reaction was also governed by both reaction time and quality of the product. We found that THF was an excellent solvent for this reaction. The main reasons for this observation is that a rather small volume is required, and that the quality of the 2-{2-[4-[(4-chlorophenyl)phenylmethyl]-1-piperazinyl]ethoxy}acetaldehyde diethylacetal produced, is surprisingly high. As proton acceptor we have chosen sodium hydride, as this base is both easy to handle and very cheap. Thus, by using the above mentioned set of reactants, a yield of 2-{2-[4-[(4-chlorophenyl)phenylmethyl]-1-piperazinyl]ethoxy}acetaldehyde diethylacetal above 95 % with a HPLC purity of about 98 % can easily be accomplished. Even more surprisingly, no trace of an N-alkylated product can be detected on HPLC.

The next step starts with the hydrolysis of 2-{2-[4-[(4-chlorophenyl)phenylmethyl]-1-piperazinyl]ethoxy}acetaldehyde diethylacetal. This can be accomplished in many ways (Theodora W. Greene, PROTECTIVE GROUPS IN ORGANIC SYNTHESIS, second edition, John Wiley & Sons, Inc. pp 178-210), but the most convenient way in this context is simply to hydrolyse in hot dilute hydrochloric acid. We have found that at 60 °C this is both a very fast and clean reaction, thereby producing 2-{2-[4-{(4-chlorophenyl)phenylmethyl]-1-piperazinyl]ethoxy}acetaldehyde dihydrochloride in quantitative yield. The hydrolysis can, however, be accomplished at lower temperatures, down to 0 °C or below, as well as at higher temperatures, up to the boiling point of the solution, thereby only increasing or reducing the reaction time required. The preferred temperatures for practical reasons are in the range of 50-80 °C, more preferably about 60 °C.

The aldehyde then needs only to be oxidised, and again there are several ways of accomplishing this (Milos Hudlicky, OXIDATIONS IN ORGANIC CHEMISTRY, American Chemical Society, Washington, D.C., 1990). In view of the demands for ecological sound procedures in large scale chemical processes, we do not wish to use oxidizing agents which could imply environmental problems. Thus, although reagents based on chromium, manganese, nickel, selenium etc are known to effect oxidation of an aldehyde to the corresponding acid in high yield, we prefer to use hydrogen peroxide for this conversion. The main reasons for this choice are that hydrogen peroxide is very cheap and that it only gives water when reduced. Of course, any peroxide could in principle be used. However, we have tried to use m-chloroperoxybenzoic acid and oxygen as oxidants, but found that hydrogen peroxide is a far superior oxidant for this aldehyde.

Surprisingly, with hydrogen peroxide, the conversion takes place under very mild conditions when the suspension is buffered (preferably in the pH range of 4 to 8). Conducting the oxidation at a pH below 4 requires a fairly long reaction time and, in addition thereto, rather large amounts of 2-{4-[(4-chlorophenyl)phenylmethyl]-1-piperazinyl}ethanol is produced during the reaction. We believe that this is a consequence of α-hydroxylation of the aldehyde (via its enol form) thereby producing a hemiacetal which is subsequently hydrolysed *in situ* to 2-{4-[(4-chlorophenyl)phenylmethyl]-1-piperazinyl}ethanol. The oxidation of the aldehyde to the acid is very fast at pH > 7, but, not surprisingly, it results in large amounts of N-oxide formation. The N-oxide has to be reduced *in situ* as it is somewhat difficult to separate from the 2-{2-[4-[(4-chlorophenyl)phenylmethyl]-1-piperazinyl]ethoxy}acetic acid formed. The pH of the reaction is usually maintained at appr. 6,5 by addition of concentrated sodium hydroxide solution, but the intervals of addition can be reduced if a buffer is present, e.g. acetic acid/acetate buffer.

The present invention will probably be better understood from the following examples which only serve to illustrate the invention and therefore should not be taken to limit the scope thereof.

### EXAMPLES

### Example 1

### A. 2-{2-[4-[(4-chlorophenyl)phenylmethyl]-1-piperazinyl]ethoxy}acetaldehyde diethylacetal

To a stirred solution of 2-{4-[(4-chlorophenyl)phenylmethyl]-1-piperazinyl}ethanol (50.9 g, 0.154 mol) in 200 ml dry THF under dry nitrogen was added sodium hydride (6 g 80% in mineral oil, 0.20 mol). The temperature was raised to 50 °C and the solution was stirred for 20 minutes whereafter bromoacetaldehyde diethylacetal (35.5 g, 0.18 mol) was added. The bath temperature was raised to 90 °C and the reaction left for 5 hours. Additional sodium hydride (1 g, 0.033 mol) and bromoacetaldehyde diethylacetal (3 g, 0.015 mol) was added, and the solution was left for another 5 hours. The reaction mixture was quenched with water (a total of 40 ml was used) and hexane (100 ml) was added in order to ease the separation. The organic phase was isolated, washed with water (40 ml) and brine/water (1:1, 40 ml). The combined aqueous solutions were extracted with toluene (20 ml) and the toluene extract was added to the THF/hexane solution. The combined organic phases were washed with brine (40 ml), filtered and evaporated *in vacuo*, ending up with a pressure of 0.5 mbar and a temperature of 70 °C. The yield of crude material was 68 g, but this material also contains mineral oil from the sodium hydride (1.4 g), thereby reducing the yield to 66.6 g (96.7 %). HPLC (nucleosil C18, MeOH/H₂O 9:1, buffer: phosphate buffer pH 7, flow = 0.9 ml/min, 230 nm), Rf = 7.94 (98.2 %). ¹H NMR (CDCl₃, 250 MHz) δ ppm: 7.2 (m, 9H); 4.6 (t, 1H); 4.2 (s, 1H); 6.6 (m,8H); 2.5 (m, 10 H); 1.2 (t, 6H).

### B. 2-{2-[4-[(4-chlorophenyl)phenylmethyl]-1-piperazinyl]ethoxy}acetic acid

2-{2-[4-[(4-chlorophenyl)phenylmethyl]-1-piperazinyl]-ethoxy}acetaldehyde diethylacetal (18.2 g of the crude product from A above, 0.0413 mol), water (20 ml) and conc. hydrochloric acid (8.3 ml, 0.1 mol) were mixed, heated for 30 min at 60 °C and then cooled to room temperature. Ethanol (20 ml) was added, and the solution was brought to pH ca. 7 with conc. sodium hydroxide solution (appr. 7 ml). To the cooled suspension (20 °C) hydrogenperoxide (4.0 ml 35 %, 0.041 mol) was added, and the resulting suspension was stirred at room temperature keeping the pH of the solution at appr. 6.5 by addition of concentrated sodium hydroxide solution. The reaction was monitored by means of HPLC (nucleosil C18, MeOH/H₂0 9:1, buffer: phosphate buffer pH 7, flow = 0.9 ml/min, 230 nm), and when the aldehyde (Rf = 5.39) was almost consumed, pH was adjusted to 9 with concentrated sodium hydroxide solution and the reaction was freed of peroxide with sodium dithionite (1.0 g, 0.0057 mol). The ethanol was evaporated *in vacuo* leaving an aqueous suspension which was diluted with water (250 ml). pH was adjusted to 9.5 with concentrated sodium hydroxide solution and the aqueous solution was extracted with butyl acetate/THF 5:1 (2 x 40 ml) at 50 °C. pH was adjusted to 1.5 with concentrated hydrochloric acid and the aqueous solution was extracted at 50 °C with butyl acetate (2 x 30 ml) and finally with cyclohexane (40 ml). The aqueous solution was cooled to room temperature, pH adjusted to 4.5 with concentrated sodium hydroxide solution, and the solution was extracted with dichloromethane (2 x 70 ml). The combined organic phases were washed with water (20 ml), dried (MgSO₄) and evaporated *in vacuo* leaving a colourless foam (14.4 g). The foam was taken up in toluene (75 ml), heated to appr. 70 °C, and cyclohexane (25 ml) was added. Cooling overnight caused crystallisation, and the formed solid was filtered off and washed with toluene/cyclohexane 1:1 (40 ml). After drying *in vacuo* 13.7 g (85.3 %) of 2-{2-[4-[(4-chlorophenyl)phenylmethyl]-1-piperazinyl]-ethoxy}acetic acid was obtained. M. P. 135-138 °C. ¹H NMR (CDCl₃, 300 MHz) δ ppm: 7.1 (m, 9H); 4.20 (s, 1H); 3.95 (s, 2H); 3.70 (m, 2H); 3.00 (s, 4 H); 2.85 (m, 2H); 2.60 (s, 4H).

### C. 2-{2-[4-[(4-chlorophenyl)phenylmethyl]-1-piperazinyl]ethoxy}acetic acid dihydrochloride

2-{2-[4-[(4-chlorophenyl)phenylmethyl]-1-piperazinyl]-ethoxy}acetic acid from B above (13.4 g, 0.034 mol) was suspended in acetone (150 ml), heated to 50 °C and concentrated hydrochloric acid (6.2 ml, 0.078 mol) was added with vigorous stirring. The resulting almost clear solution was stirred at room temperature for 2 hours after which a fine white precipitate of 2-{2-[4-[(4-chlorophenyl)phenylmethyl]-1-piperazinyl]ethoxy}acetic acid dihydrochloride had formed. The solution was filtered, washed with acetone (50 ml) and dried *in vacuo* leaving 13.8 g (87.9 %) of 2-{2-[4-[(4-chlorophenyl)phenylmethyl]-1-piperazinyl]ethoxy}acetic acid dihydrochloride. M. P. 224-226 °C.

### Example 2

### 2-{2-[4-[(4-chlorophenyl)phenylmethyl]-1-piperazinyl]-ethoxy}acetaldehyde dihydrochloride

In order to characterize the above intermediate concentrated hydrochloric acid (1 ml, 0,0125 mol) and water (4 ml) was added to 2-{2-[4-[(4-chlorophenyl)phenylmethyl]-1-piperazinyl]ethoxy}acetaldehyde diethylacetal (2 g of the crude product obtained under A in Example 1, 0.0045 mol), and the mixture was heated for 20 minutes at 50 °C. Acetone (20 ml) was added, and the mixture was treated with active carbon, heated to reflux and filtered. The solution was evaporated *in vacuo* until a colourless foam appeared. Acetone (50 ml) was added, and the suspension was stirred for two hours, after which a colourless precipitate had formed. This was filtered off, washed with acetone and dried *in vacuo*. The yield of 2-{2-[4-[(4-chlorophenyl)phenylmethyl]-1-piperazinyl]ethoxy}acetaldehyde dihydrochloride was 1.32 g (78 %).

## Claims

1. A process for the manufacture of 2-{2-[4-(diphenylmethyl)-1-piperazinyl]ethoxy}acetic acid compounds of the general formula I: 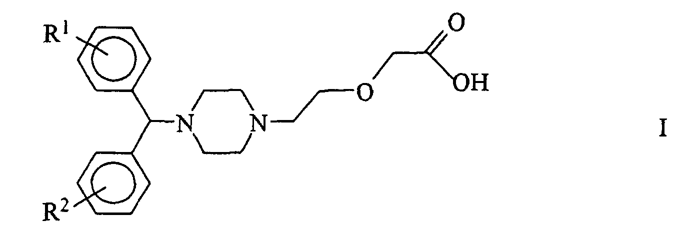 wherein R¹ and R² independently represent a hydrogen atom, a halogen atom or a trifluoromethyl radical, or salts thereof, **characterized by** reacting a 2-[4-(diphenylmethyl)-1-piperazinyl]ethanol having the formula II: 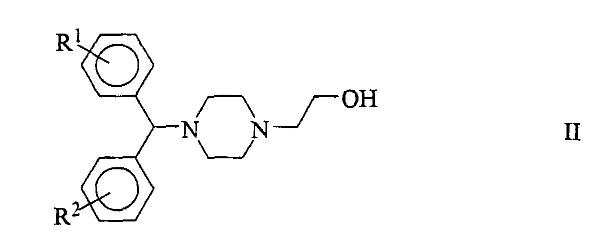 wherein R¹ and R² are as defined above, with a 2-substituted acetaldehyde dialkylacetal having the formula III: 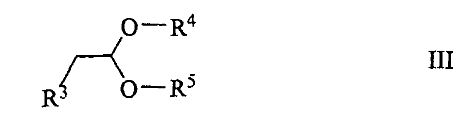 wherein R³ represents a leaving group; and R⁴ and R⁵ independently represent an alkyl radical of 1-4 carbon atoms, or R⁴ and R⁵ together form an alkylene chain of 2-4 carbon atoms, in the presence of a proton acceptor in an inert solvent to form a diphenylmethylpiperazinoethoxyacetaldehyde dialkylacetal having the formula IV: 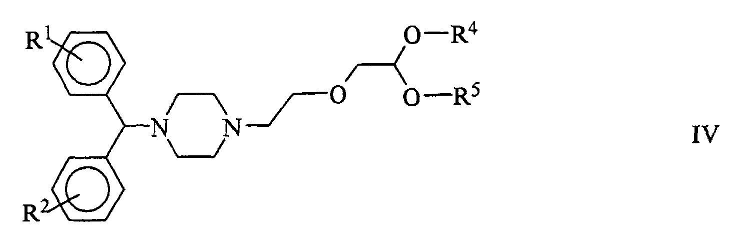 wherein R¹, R², R⁴ and R⁵ are as defined above, and thereafter hydrolysing the acetal (IV) to the corresponding aldehyde, catalysed by a proton donor, and then oxidising the aldehyde to the acid (I) by means of a suitable oxidation agent, where after, if desired, the acid (I) is converted into a salt thereof.

2. A process according to claim 1, **characterized in that** R¹ is 4-chloro and R² is hydrogen.

3. A process according to claim 1 or 2, **characterized in that** R³ is Cl, Br, I, or a sulfuric ester group.

4. A process according to claim 3, **characterized in that** R³ is Br.

5. A process according to any one of claims 1-4, **characterized in that** R⁴ and R⁵ are independently selected among methyl, ethyl, propyl, isopropyl, butyl, isobutyl and tert-butyl.

6. A process according to claim 5, **characterized in that** R⁴ and R⁵ are both ethyl.

7. A process according to any one of claims 1-6, **characterized in that** the proton acceptor is selected among alkali metal hydrides, alkalimetal hydroxides, alkali metal alkoxides and alkali metals.

8. A process according to claim 7, **characterized in that** the proton acceptor is sodium hydride.

9. A process according to any one of claims 1-8, **characterized in that** the inert solvent is selected among aliphatic and aromatic hydrocarbons, ethers, amides and alcohols.

10. A process according to claim 9, **characterized in that** the inert solvent is hexane, toluene, dimethoxyethane (DME), tetrahydrofurane (THF), dimethylformamide (DMF) or tert-butanol.

11. A process according to any one of claims 1-10, **characterized in that** the proton donor is hydrochloric acid or formic acid

12. A process according to any one of claims 1-11, **characterized in that** the hydrolysis of the acetal (IV) is carried out in dilute hydrochloric acid.

13. A process according to claim 12, **characterized in that** the hydrolysis is carried out at a temperature of 0-100 °C, more preferably 50-80 °C, and most preferably about 60 °C.

14. A process according to any one of claims 1-13, **characterized in that** the oxidation of the aldehyde is carried out by means of an oxidation agent selected among reagents based on metals, oxygen, oxo acids of halogens, peroxides and peracids.

15. A process according to claim 14, **characterized in that** the oxidation is carried out by means of hydrogen peroxide in an aqueous suspension buffered at a pH in the range of 4 to 8.

16. A 2-{2-[4-(diphenylmethyl)-1-piperazinyl]ethoxy}acetaldehyde dialkylacetal compound having the formula IV: 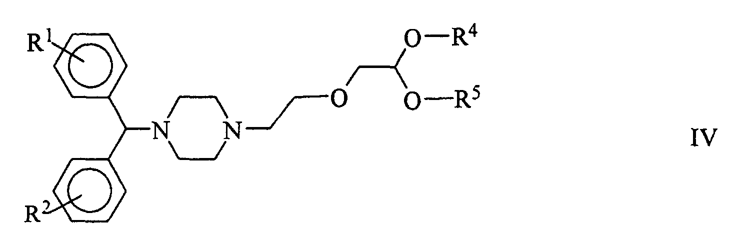 wherein R¹ and R² independently represent a hydrogen atom, a halogen atom or a trifluoromethyl radical, and R⁴ and R⁵ independently represent an alkyl radical of 1-4 carbon atoms, or R₄ and R₅ together form an alkylene chain of 2-4 carbon atoms, or a salt thereof.

17. A 2-{2-[4-(diphenylmethyl)-1-piperazinyl]ethoxy}acetaldehyde compound having the formula IVa: 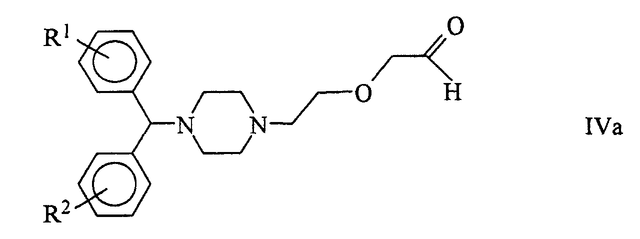 wherein R¹ and R² independently represent a hydrogen atom, a halogen atom or a trifluoromethyl radical, or a salt thereof.

## Patentansprüche

1. Verfahren für die Herstellung von 2-{2-[4-(Diphenylmethyl)-1-piperazinyl]ethoxy}essigsäure-Verbindungen der allgemeinen Formel I 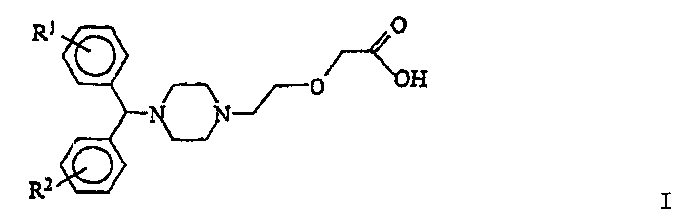 wobei R¹ und R² unabhängig voneinander ein Wasserstoffatom, ein Halogenatom oder ein Trifluormethyl-Radikal oder Salze derselben darstellen, **gekennzeichnet durch** Reagieren eines 2-[4-(Diphenylmethyl)-1-piperazinyl]ethanols der Formel II: 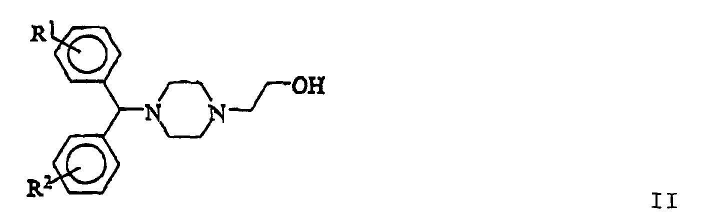 wobei R¹ und R² die oben angegebene Definition aufweisen, mit einem 2-substituierten Acetaldehyddialkylacetal der Formel III: 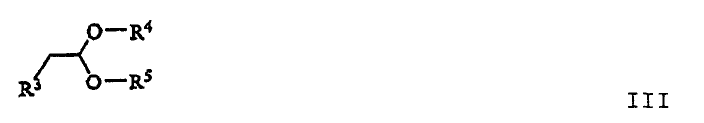 wobei R³ eine Austrittsgruppe und R⁴ und R⁵ unabhängig voneinander ein Alkylradikal mit 1 - 4 Kohlenstoffatomen darstellen oder R⁴ und R⁵ zusammen eine Alkylenkette mit 2 - 4 Kohlenstoffatomen bilden, in Anwesenheit eines Protonenakzeptors in einem inerten Lösungsmittel unter Bildung eines Diphenylmethylpiperazinethoxyacetaldehyddialkylacetals der Formel IV 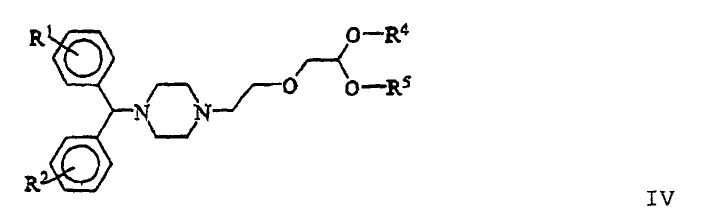 wobei R¹, R², R⁴ und R⁵ die oben angegebene Definition aufweisen, und darauffolgendes Hydrolysieren des Acetals (IV) zum entsprechenden Aldehyd, **durch** einen Protonendonator katalysiert, und darauffolgendes Oxidieren des Aldehyds zur Säure (I) **durch** ein geeignetes Oxidationsmittel, woraufhin die Säure (I), falls erwünscht, in ein Salz derselben umgewandelt wird.

2. Verfahren nach Anspruch 1, **dadurch gekennzeichnet, dass** R¹ für 4-Chloro und R² für Wasserstoff steht.

3. Verfahren nach Anspruch 1 oder 2, **dadurch gekennzeichnet, dass** R³ für Cl, Br, I oder eine Schwefelestergruppe steht.

4. Verfahren nach Anspruch 3, **dadurch gekennzeichnet, dass** R³ für Br steht.

5. Verfahren nach einem der Ansprüche 1 - 4, **dadurch gekennzeichnet, dass** R⁴ und R⁵ unabhängig voneinander unter Methyl, Ethyl, Propyl, Isopropyl, Butyl, Isobutyl und tert.-Butyl ausgewählt sind.

6. Verfahren nach Anspruch 5, **dadurch gekennzeichnet, dass** R⁴ und R⁵ beide für Ethyl stehen.

7. Verfahren nach einem der Ansprüche 1 - 6, **dadurch gekennzeichnet, dass** der Protonenakzeptor unter Alkalimetallhydriden, Alkalimetallhydroxiden, Alkalimetallalkoxiden und Alkalimetallen ausgewählt wird.

8. Verfahren nach Anspruch 7, **dadurch gekennzeichnet, dass** der Protonenakzeptor Natriumhydrid ist.

9. Verfahren nach einem der Ansprüche 1 - 8, **dadurch gekennzeichnet, dass** das inerte Lösungsmittel unter aliphatischen und aromatischen Kohlenwasserstoffen, Ethern, Amiden und Alkoholen ausgewählt ist.

10. Verfahren nach Anspruch 9, **dadurch gekennzeichnet, dass** das inerte Lösungsmittel Hexan, Toluol, Dimethoxyethan (DME), Tetrahydrofuran (THF), Dimethylformamid (DMF) oder tert.-Butanol ist.

11. Verfahren nach einem der Ansprüche 1 - 10, **dadurch gekennzeichnet, dass** der Protonendonator Salzsäure oder Ameisensäure ist.

12. Verfahren nach einem der Ansprüche 1 - 11, **dadurch gekennzeichnet, dass** die Hydrolyse des Acetals (IV) in verdünnter Salzsäure durchgeführt wird.

13. Verfahren nach Anspruch 12, **dadurch gekennzeichnet, dass** die Hydrolyse bei einer Temperatur von 0 - 100 °C, noch bevorzugter von 50 - 80 °C und am bevorzugtesten bei ca. 60 °C durchgeführt wird.

14. Verfahren nach einem der Ansprüche 1 - 13, **dadurch gekennzeichnet, dass** die Oxidation des Aldehyds mit Hilfe eines Oxidationsmittels durchgeführt wird, das unter Reagenzien auf der Basis von Metallen, Sauerstoff, Oxosäuren von Halogenen, Peroxiden und Persäuren ausgewählt wird.

15. Verfahren nach Anspruch 14, **dadurch gekennzeichnet, dass** die Oxidation mit Hilfe von Wasserstoffperoxid in einer wässrigen Suspension durchgeführt wird, die auf einen pH-Wert im Bereich von 4 bis 8 gepuffert ist.

16. 2-{2-[4-(Diphenylmethyl)-1-piperazinyl]ethoxy}-acetaldehyddialkylacetat-Verbindungen der Formel IV 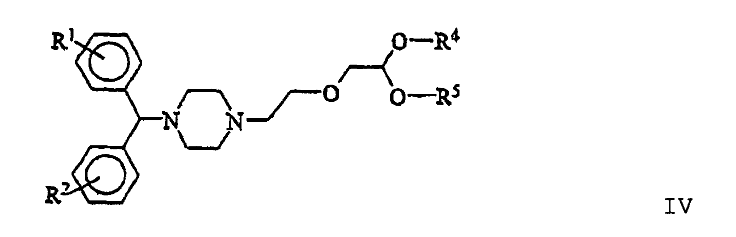 wobei R¹ und R² unabhängig voneinander ein Wasserstoffatom, ein Halogenatom oder ein Trifluormethyl-Radikal und R⁴ und R⁵ unabhängig voneinander ein Alkylradikal mit 1 - 4 Kohlenstoffatomen oder R⁴ und R⁵ zusammen eine Alkylenkette mit 2 - 4 Kohlenstoffatomen oder ein Salz derselben darstellen.

17. 2-{2-[4-(Diphenylmethyl)-1-piperazinyl]ethoxy}-acetaldehyd-Verbindungen der Formel IVa 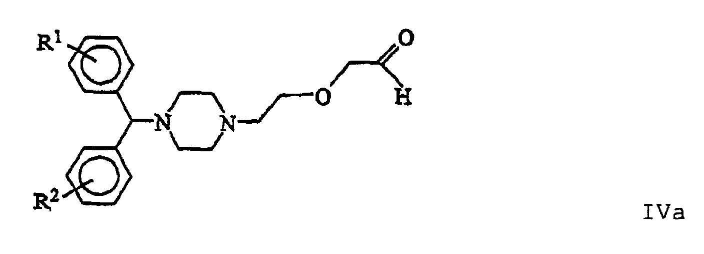 wobei R¹ und R² unabhängig voneinander ein Wasserstoffatom, ein Halogenatom oder ein Trifluormethyl-Radikal oder ein Salz derselben darstellen.

## Revendications

1. Processus pour la fabrication des composés d'acide 2-{2-[4-(diphénylméthyl)-1-pipérazinyl]éthoxy} acétique de formule générale I : 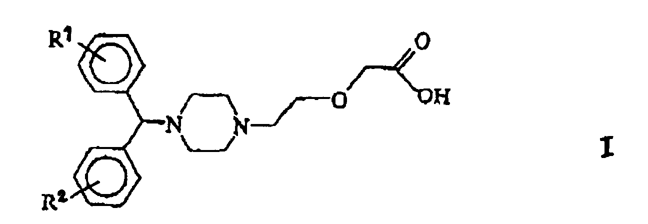 dans laquelle R¹ et R² représentent indépendamment un atome d'hydrogène, un atome d'halogène ou un radical trifluorométhyle, ou leurs sels, **caractérisé par** la réaction d'un 2-[4-(diphénylméthyl)-1-pipérazinyl]éthanol ayant la formule II : 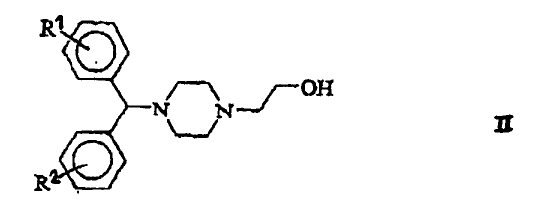 dans laquelle R¹ et R² sont tels que définis ci-dessus, avec un acétaldéhyde dialkylacétal 2-substitué ayant la formule III : 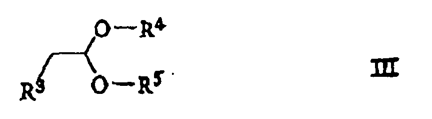 dans laquelle R³ représente un groupe partant ; et R⁴ et R⁵ représentent indépendamment un radical alkyle de 1 à 4 atomes de carbone, ou R⁴ et R⁵ forment ensemble une chaîne alkylène de 2 à 4 atomes de carbone, en présence d'un accepteur de proton dans un solvant inerte pour former un diphénylméthylpipérazinoéthoxyacétaldéhyde dialkylacétal ayant la formule IV : 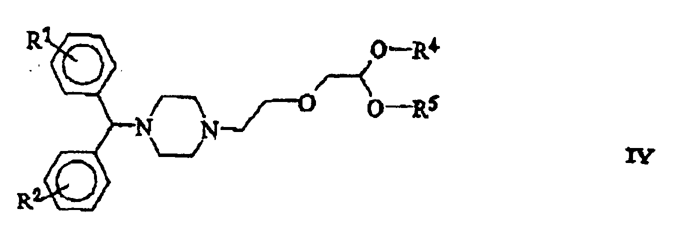 dans laquelle R¹, R², R⁴ et R⁵ sont tels que définis ci-dessus, puis l'hydrolyse de l'acétal (IV) en l'aldéhyde correspondant, catalysée par un donneur de proton, puis l'oxydation de l'aldéhyde en l'acide (I) au moyen d'un agent d'oxydation adapté, où ensuite, si on le souhaite, l'acide (I) est converti en un sel de celui-ci.

2. Processus selon la revendication 1, **caractérisé en ce que** R¹ est le 4-chloro et R² est l'hydrogène.

3. Processus selon la revendication 1 ou 2, **caractérisé en ce que** R³ est Cl, Br, I ou un groupe ester sulfurique.

4. Processus selon la revendication 3, **caractérisé en ce que** R³ est Br.

5. Processus selon l'une quelconque de revendications 1 à 4, **caractérisé en ce que** R⁴ et R⁵ sont choisis indépendamment parmi le méthyle, l'éthyle, le propyle, l'isopropyle le butyle, l'isobutyle et le tert-butyle.

6. Processus selon la revendication 5, **caractérisé en ce que** R⁴ et R⁵ sont tous les deux un éthyle.

7. Processus selon l'une quelconque de revendications 1 à 6, **caractérisé en ce que** l'accepteur de proton est choisi parmi les hydrures de métal alcalin, les hydroxydes de métal alcalin, les alcoxydes de métal alcalin et les métaux alcalins.

8. Processus selon la revendication 7, **caractérisé en ce que** l'accepteur de proton est l'hydrure de sodium.

9. Processus selon l'une quelconque de revendications 1 à 8, **caractérisé en ce que** le solvant inerte est choisi parmi les hydrocarbures aliphatiques et aromatiques, les éthers, les amides et les alcools.

10. Processus selon la revendication 9, **caractérisé en ce que** le solvant inerte est l'hexane, le toluène, le diméthoxyéthane (DME), le tétrahydrofurane (THF), le diméthylformamide (DMF) ou le tert-butanol.

11. Processus selon l'une quelconque de revendications 1 à 10, **caractérisé en ce que** le donneur de proton est l'acide chlorhydrique ou l'acide formique.

12. Processus selon l'une quelconque de revendications 1 à 11, **caractérisé en ce que** l'hydrolyse de l'acétal (IV) est effectuée dans l'acide chlorhydrique dilué.

13. Processus selon la revendication 12, **caractérisé en ce que** l'hydrolyse est effectuée à une température de 0 à 100°C, de manière davantage préférée de 50 à 80°C, et de manière préférée entre toutes à environ 60°C.

14. Processus selon l'une quelconque de revendications 1 à 13, **caractérisé en ce que** l'oxydation de l'aldéhyde est effectuée au moyen d'un agent d'oxydation choisi parmi les réactifs basés sur les métaux, l'oxygène, les acides oxo d'halogènes, les peroxydes et les peracides.

15. Processus selon la revendication 14, **caractérisé en ce que** l'oxydation est effectuée au moyen du peroxyde d'hydrogène dans une suspension aqueuse tamponnée à un pH dans la plage de 4 à 8.

16. Composé 2-{2-[4-(diphénylméthyl)-1-pipérazinyl]éthoxy}-acétaldéhyde dialkylacétal ayant la formule IV : 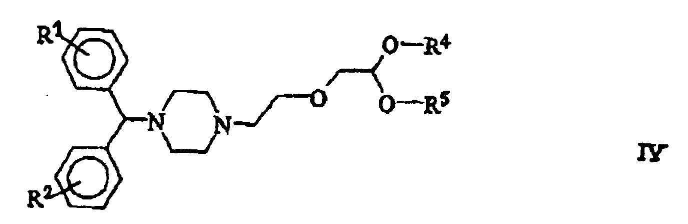 dans laquelle R¹ et R² représentent indépendamment un atome d'hydrogène, un atome d'halogène ou un radical trifluorométhyle, et R⁴ et R⁵ représentent indépendamment un radical alkyle de 1 à 4 atomes de carbone, ou R⁴ et R⁵ ensemble forment une chaîne alkylène de 2 à 4 atomes de carbone, ou un de leurs sels.

17. Composé 2-{2-[4-(diphénylméthyl)-1-pipérazinyl]éthoxy}-acétaldéhyde ayant la formule IVa : 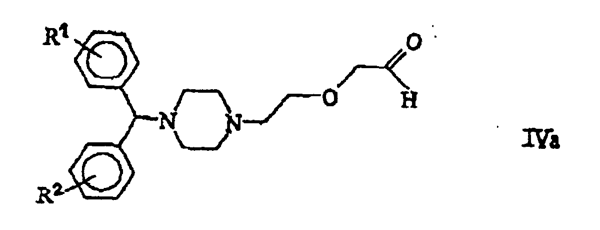 dans laquelle R¹ et R² représentent indépendamment un atome d'hydrogène, un atome d'halogène ou un radical trifluorométhyle, ou un de leurs sels.
